Europäisches Patentamt

European Patent Office

Office européen des brevets

(19)

(11) Publication number: **0 294 846**

. **A2**

(12) # EUROPEAN PATENT APPLICATION

(21) Application number: **88109313.2**

(22) Date of filing: **10.06.88**

(51) Int. Cl.⁴: **C07C 51/235 , C07C 53/08**

The title of the invention has been amended (Guidelines for Examination in the EPO, A-III, 7.3).

(30) Priority: **12.06.87 US 61151**

(43) Date of publication of application:
**14.12.88 Bulletin 88/50**

(84) Designated Contracting States:
**BE DE FR GB IT LU NL SE**

(71) Applicant: **UNION CARBIDE CORPORATION**
**39 Old Ridgebury Road**
**Danbury Connecticut 06817(US)**

(72) Inventor: **McCain, James Herndon, Jr.**
**1987 Parkwood Road**
**Charleston 25314 Virginia(US)**
Inventor: **Kaiser, Steven William**
**900 Glendale Avenue**
**South Charleston 25303 Virginia(US)**

(74) Representative: **Wuesthoff, Franz, Dr.-Ing. et al**
**Patentanwälte Wuesthoff -v.**
**Pechmann-Behrens-Goetz Schweigerstrasse**
**2**
**D-8000 München 90(DE)**

(54) **Preparation of organic acids from alkanols.**

(57) A process for the production of an organic acid by the catalytic oxidation of an alcohol in contact with a calcined oxides catalyst of the composition:

$$Mo_x V_y Z_z$$

in which Z represents nothing or a metal from a given group of metals.

EP 0 294 846 A2

## ORGANIC ACIDS FROM ALKANOLS

Field of Invention

This invention relates to the selective production of an organic acid, e.g. acetic acid, by the catalytic oxidation of an alcohol with oxygen, e.g. ethanol, in contact with a calcined oxide catalyst containing the metals Mo and V, alone or with at least one other metal.

Description of Prior Art

U.S.-A-4,220,803 discloses a process for the vapor phase oxidative dehydrogenation of ethanol to produce acetic acid and acetaldehyde using a supported copper oxide catalyst that is essentially free of barium. The patent makes note of known processes and references and the problems associated with the prior attempts to produce acetic acid from ethanol, formation of acetaldehyde being favored over formation of the acid.

U.S.-A-4,250,346 discloses a process for the oxydehydrogenation of ethane to ethylene using a calcined catalyst containing the elements $Mo_a\,X_b\,Y_c$ in which X can be one or more of V, Nb and Mn, V and W, V and Mn or W and Nb; Y is Bi, Ce, Co, Cu, Fe, K, Mg, Ni, P, Pb, Sb, Si, Sn, Tl and/or U and (a), (b) and (c) are as defined. Though some acetic acid is produced as a by-product, the starting material charged to the reactor is ethane and the desired product selectively produced is ethylene, both of which differ from the invention described in this instant application.

U.A.-A-4,524,236 discloses the use of an calcined catalyst of the formula $Mo_aV_bNb_cSb_dX_e$ for the oxydehydrogenation of ethane to ethylene with some acetic acid being formed as a by-product. Among the catalysts disclosed are the catalysts used in this instant application for preparing acetic acid from ethanol and oxygen. The process described in the patent, however, is not the process of this application, nor does the patent suggest the process of this invention.

U.S.-A-4,568,790 discloses a process for the oxydehydrogenation of ethane to ethylene using a calcined catalyst containing $Mo_aV_bNb_cSb_d$.

The chemical process involved is not the same as is in this instant application.

The last three patents discussed above also make reference to other patents concerned with the production of ethylene from ethane using a wide variety of catalyst compositions. However, none of these additional patents suggest or disclose the process of this application, namely, the selective production of organic acids, e.g. acetic acid, from alkanols, e.g. ethanol, by an oxidation reaction.

Japan 57-102835 is concerned with a process for producing acetic acid from ethanol or acetaldehyde using a catalyst containing a copper oxide. The reference does not suggest or disclose our catalyst.

Japan 54-57488 is concerned with the production of acetic acid by the oxidation of ethylene using a palladium modified molybdovanadophosphoric acid catalyst of the structure $[NaPd_{0.5}H_2(PMo_{11}VO_{40})]$. Not only does the catalyst differ from our catalyst but the reference does not disclose the production of organic acids from alkanols.

Japan 46-6763 is concerned with the catalytic oxidation of ethylene to yield mixtures of acetic acid, formic acid, acetaldehyde and carbon dioxide and other unidentified products. The specific catalysts disclosed are not those of the instant application and the patent does not disclose the production of organic acids from alkanols.

E. M. Thorsteinson, T. P. Wilson, F. G. Young and P. H. Kasai in "The Oxidative Dehydrogenation of Ethane Over Catalysts Containing Mixed Oxides of Molybdenum and Vanadium", J. Catal, 52, 116-132 (1978), describe the oxydehydrogenation of ethane to ethylene using MoVNb oxides catalyst systems. The article does mention the formation of acetic acid and other by-products formed during the oxydehydrogenation reaction. Further, on page 122, in the last full paragraph in the second column, the authors mention passing ethanol over the catalyst "without added oxygen" and obtaining ethane, ethylene, acetic acid, acetaldehyde, carbon dioxide, carbon monoxide and water as the products. They do not disclose an oxidation reaction that co-feeds oxygen to the reactor along with the alkanol, which is applicants' process.

In a recent article "Catalytic Oxidation of Ethanol to Acetic Acid in Gas Phase", T.G. Alkhazov et al, Perv. Sov.-Indiisk. Seminar po Katalizu na Temu: Kataliz i Progress v. Khim Tekhnol, 99-103 (1984), a molybdenum oxide catalyst is used to produce acetic acid from ethanol. However, the composition of the catalyst is nowhere discussed.

## Summary of the Invention

The present invention relates to a process for the catalytic oxidation of an alcohol with oxygen to selectively produce its corresponding acid, for example, ethanol to acetic acid. In the process of this invention a catalyst containing molybdenum and vanadium and which can contain at least one other metal atom as represented by the general formula $Mo_xV_yZ_z$ in which the metal elements are in combination with oxygen in the form of various oxides is used. One can also optionally have present in the reaction mixture any of the known ethylene oxidation catalysts or ethylene hydration catalysts, all of which are well known to those of ordinary skill in the art. In this general formula Z can be nothing or one or more of Li, Na, Be, Mg, Ca, Sr, Ba, Zn, Cd, Hg, Sc, Y, La, Ce, Al, Tl, Ti, Zr, Hf, Pb, Nb, Ta, As, Sb, Bi, Cr, W, U, Te, Fe, Co, Ni and x is equal to 0.5 to 0.9, y is equal to 0.1 to 0.4 and z is equal to 0 to 1. Many of these catalysts are known as shown by the prior art previously discussed and include compositions of the combinations of metals such as MoV, MoVNb, MoVSb, MoVNbSb, MoVCa, MoVNbSbCa, MoVNbSbSr, MoVNbSbNa, MoVNbSbBa, MoVNbSbMg, MoVNbSbFe, MoVNbSbCaK, and the like, shown without subscripts (x), (y) and (z).

The preferred catalysts for use in the process of this invention are the calcined oxides compositions of the formula $Mo_a\,V_b\,Nb_c\,Sb_d\,X_e$ wherein X is at least one of the following metals Li, Na, Be, Mg, Ca, Sr, Ba, Zn, Cd, Hg, Sc, Y, La, Ce, Al, Tl, Ti, Zr, Hf, Pb, Ta, As, Bi, Cr, W, U, Te, Fe, Co and Ni , preferably Ca; and a is equal to 0.5 to 0.9, b is equal to 0.1 to 0.4, c is equal to 0.001 to 0.2, d is equal to 0.001 to 0.1 and e is equal to 0.001 to 1.0. The values of a , b , c , d and e constitute relative gram atoms of the respective elements in the catalyst. The elements are present in combination with oxygen in the form of various oxides.

## Detailed Description of the Invention

The catalytic oxidation of alcohols to acids and other compounds is known. However, among the problems associated with the known processes is the low selectivity to acid attained; for instance in the production of acetic acid from ethanol. Consequently, improvements are constantly being sought that will decrease by-product formation and increase acetic acid selectivity plus other products of value, e.g., ethylene, rather than the undesirable oxides of carbon. Though many catalytic oxidation processes are known, including processes that employ molybdenum-containing catalysts, an improvement in selectivity to the acid is commercially desirable since it permits acetic acid production from ethanol derived from grain rather than derived from natural gas and oil.

In the process of this invention an aliphatic alcohol of the formula ROH, wherein R is a linear or branched alkyl group having from 2 to 10 carbon atoms, preferably 2 to 4 carbon atoms and most preferably 2, carbon atoms, is catalytically oxidized with oxygen in the vapor phase using a calcined oxides catalyst composition of the general formula:

(I)     $Mo_xV_yZ_z$

and preferably of the general formula:

(II)     $Mo_a\,V_b\,Nb_c\,Sb_d\,X_e$

as hereinbefore defined. As previously indicated an ethylene oxidation catalyst and/or an ethylene hydration catalyst can also be present. Generally, the catalyst composition contains more than about 40g atom percent molybdenum and more than about 2 g atom percent vanadium. The process of this invention yields acetic acid at an unexpected and unpredictable high selectivity. It was further found that the addition of ethane to the vapor feed resulted in unexpected and unpredictable high selectivity to the acid and olefin. For example oxidation of a vapor phase mixture of oxygen, ethanol and ethane with the defined calcined oxide catalyst composition of Formula II resulted in selectivity to acetic acid plus ethylene as high as 96 mole percent. The ethylene produced is an important product since it could then be readily converted to ethanol by known hydration processes and recycled to produce additional quantities of acetic acid; or it could be recovered and used in other known chemical processes, ethylene being a valuable chemical commodity.

The catalysts employed in the process of this invention can be used with or without a support. Their preparations are well-known and are fully described in the prior art, e.g., U.S. - A - 4,524,236, U.S. - A - 4,250,346 and U.S. - A - 4,568,790, previously mentioned. They have the compositions stated in the section entitled "Summary of the Invention", with the preferred catalyst being the calcined oxides composition $Mo_aV_bNb_cSb_dCa_e$.

Preferably, the catalysts are prepared from a solution of soluble compounds and/or complexes and/or compounds of each of the metals. The solution is preferably an aqueous system having a pH of 1 to 12 and more preferably a pH of 5 ± 3, at a temperature of from 20°C to 100°C.

Generally, a mixture of compounds containing the selected metal elements is prepared by dissolving sufficient quantities of soluble compounds of those metals and dispersing the insoluble compounds so as to provide the desired gram-atom ratios of the metal elements in the catalyst composition. The catalyst composition is then prepared by removing the water or other solvent from the mixture of the compounds in the solution system. The dried catalyst is calcined by heating to a temperature of from 220°C to 550°C in air or oxygen for a period of time from one minute to 24 hours to produce the desired catalyst composition. Generally, the higher the temperature the shorter the period of time required.

Suitable supports for the catalyst include silica, aluminum oxide, silicon carbide, zirconia, titania, and mixtures thereof. When used on a support, the supported catalyst usually comprises from 10 to 50% by weight of the catalyst composition, with the remainder being the support.

Preferably, the molybdenum is introduced into the solution in the form of ammonium salts such as ammonium paramolybdate, or organic acid salts of molybdenum such as acetates, oxalates, mandelates, and glycolates. Some other partially water soluble molybdenum compounds which may be used include molybdenum oxides, molybdic acid, and the chlorides of molybdenum.

Preferably, the vanadium is introduced into the solution in the form of ammonium salts such as ammonium meta-vanadate and ammonium decavanadate, or organic acid salts of vanadium such as acetates, oxalates, and tartrates. Partially water soluble vanadium compounds such as vanadium oxides, and sulfates of vanadium can be used.

Preferably, when it is present, the niobium is added in the form of the oxalate. Other sources of this metal in soluble form include compounds in which the metal is coordinated, bonded or complexed to a beta-diketonate, carboxylic acid, an amine, an alcohol, or an alkanolamine.

Preferably, when it is present, the antimony is introduced into solution in the form of antimony oxalate. Other soluble and insoluble compounds of antimony can be used such as antimony oxide and antimony chloride.

The Z or X component of the catalyst can be a soluble or insoluble compound preferably soluble. Compounds which are strongly reducing may adversely reduce the oxidation states of the metal.

The following are some preferable compounds for the Z or X component. One is calcium in the form of a water soluble chelate coordinated with ammonium lactate, and others are calcium compounds in which the metal is coordinated, or complexed to a beta-diketonate, a carboxylic acid, an amine, an alcohol or an alkanolamine. Generally, nitrates of the Z or X components are desirable along with water soluble chlorides and organic acid salts such as acetates, oxalates, tartrates, lactates, salicylates, formates, and carbonates.

Preferably, the catalyst is prepared by the following general procedure. The vanadium compound is mixed with water to form a first solution or suspension, the niobium and antimony are mixed with water to form a second solution or suspension, and molybdenum compound is mixed with water to form a third solution or suspension. Any Z or X compounds which are ammonium salts are mixed with the first solution. Otherwise, the Z or X compounds are mixed into the second solution. The first and second solutions are heated separately and mixed for about fifteen minutes; and then combined and mixed with heating for about fifteen minutes. The third solution is heated and mixed, and then added to the combined first and second solutions to form a combined suspension or solution. After mixing and heating of the combined mixtures for about fifteen minutes, the combined mixture is evaporated to dryness rapidly, in air usually, but the drying could be carried out in an inert atmosphere.

When the catalyst is to be used with a support, it is believed desirable to filter the combined solution to remove the insoluble portion before impregnating the support. The filtering can be carried out using sintered glass, or a paper filter with or without suction.

It has been found that catalyst surface area and activity depend on the digestion time, i.e., the time taken to evaporate the combined mixture to dryness. Compositions allowed to digest for relatively long periods of time, thirty minutes or more, before drying at 120°C. generally undergo particle growth with loss in surface area.

It is believed that the catalyst for the invention should have one or more of the metal components slightly below their highest possible oxidation states. The calcining is carried out with the flow of air or some other oxygen containing gas over the dry solids prepared from the solutions or suspensions to control the reducing actions of reducing agents such as $NH_3$ or organic reducing agents which are introduced into the solution system from which the catalysts are prepared. The rate of flow of the gas can be determined experimentally for the apparatus and the quantities of solids being used for optimizing the properties of the catalyst being produced.

One or more of the free valances of metals in the catalyst are occupied by one or more of oxide, hydroxyl, and $CO_3$.

4

In general, the catalyst, supported or unsupported can be used in a fixed or fluidized bed.

Any vaporizable aliphatic alcohol containing from 2 to 10 carbon atoms, preferably ethanol, can be used in the process. It is fed into the reactor as a gas stream together with oxygen or air and, optionally, other gases, such as methane and water, in the vapor phase. For simplicity the present process will be described using ethanol as the alcohol, it being understood that it applies to the other alcohols defined.

It has been observed that the presence of ethane in the gas feed stream improves the selectivity of ethanol to acetic acid plus ethylene. This is particularly so when the amount of ethane is greater than the molar amount of oxygen in the feed. The amount of ethane can vary from 0.1 mole to 100 moles of ethane per mole of oxygen, preferably from 0.8 mole to 50 moles of ethane per mole of oxygen and most preferably from 1.1 moles to 20 moles of ethane per mole of oxygen.

The reactor used in Examples 1-3 was a stainless steel tubular reactor measuring 1.27 cm inside diameter and 20.3 cm long. The reactor is charged with the catalyst, supported or unsupported, preferably unsupported, and not necessarily completely filled with catalyst. It was immersed in a thermostated sand bath for temperature control purposes. The outlet was equipped with known means for recovery of gaseous and liquid products. Connected to the inlet of the reactor was a 20.3 cm long by 0.7 cm inside diameter stainless steel tube packed with glass beads, which served as a preheater. The preheater was heated by immersion in the same sand bath used for the reactor. Reactants were introduced into the preheater and from thence into the reactor with reactant flow monitored with a bubble meter at the exit end of the analytical train. Uncondensed product was passed through a water condenser and led through a condensation train of wet ice and then dry ice/acetone in series so that all low boiling product was recovered. Uncondensed gases and low boiling products were analyzed by standard gas chromatograph techniques.

The reaction mixture introduced into the reactor in the process of this invention is generally in the ratio of one mole of alcohol to 1 mole to 10 moles or more of oxygen either as pure oxygen or in the form of air, and zero to 10 moles or more of water in the form of steam and zero to 10 moles or more of ethane in gaseous form. The water or steam is used as a reaction diluent and heat moderator for the reaction. The ethane is used as a reactant when it is present, and when it is present it is preferably present in the feed at a molar concentration greater than the molar concentration of oxygen charged.

The feed components are generally premixed, and preheated to the gaseous form prior to being introduced into the reaction zone. The reaction zone has a temperature of from 75°C to 500°C, preferably from 200°C to 400°C.

The pressure in the reactor can vary from atmospheric pressure to 75 atmospheres, preferably from 1 to 30 atmospheres.

A contact time of from 0.01 second to 100 seconds, preferably from 0.1 second to 10 seconds, of the reaction feed with the catalyst is maintained for the reaction. The contact time is defined as the ratio between the apparent volume of the catalyst bed and the volume of the gaseous reaction mixture feed to the catalyst bed under the given reaction conditions in a unit of time.

A space velocity in the reaction zone of from 50 to 50,000 $h^{-1}$, preferably 100 to 10,000 $h^{-1}$ and most preferably 200 to 3,000 $h^{-1}$ is maintained. The space velocity is calculated by determining total reactor outlet gas equivalent in liters of the total effluent evolved over a period of one hour divided by the liters of catalyst in the reactor. This room temperature volume is converted to the volume at 0°C at 1 bar (760 mm Hg):

$$\text{space velocity} = \frac{\text{liters of outlet gas equivalents per hour}}{\text{liters of catalyst in reactor}} = h^{-1}$$

The oxygen concentration in the feed gas mixture can vary widely, from 0.1 to 50 % oxygen or higher of the feed mixture. As previously indicated air is the preferred source of oxygen in the feed. The amount of oxygen present may be a stoichiometric amount, or less, of the alcohol in the feed, preferably, however, oxygen will be in excess.

The process is generally carried out in a single stage with all of the oxygen and reactants being supplied as a single feed. However, multiple stage addition of oxygen to the reactor with intermediate alcohol feed can also be used.

The amount of water or steam in the gaseous feed mixture will vary and 10 weight percent to 30 weight percent in the feed is preferred.

In addition to the components referred to, one can also have present in the feed amounts of other

5

compounds, e.g. diethyl ether.

The unexpected higher selectivity to acetic acid achieved by the process of this invention was completely unexpected and unpredictable. Even less predictable was the higher selectivity to acetic acid plus ethylene achieved by the catalytic oxidation of ethanol and ethane mixtures with this process.

The following examples serve to further illustrate this invention.

## Example 1

A $Mo_{16}V_{5.6}Nb_{0.5}Sb_{0.3}Ca_{0.3}$ calcined oxides catalyst was prepared by the procedure previously described using ammonium molybdate (4 g atoms of Mo), ammonium metavanadate (1.7 g atoms of V), niobium oxalate (0.47 g atom of Nb), antimony oxalate (0.25 g atom of Sb) and calcium nitrate (0.25 g atom of Ca). The compounds were thoroughly mixed in a total of 6,000 ml of water at 75°C and filtered. The filtered solution was evaporated to dryness and the solids obtained broken to 0.42 - 0.84 mm (20 to 40 mesh) particles and then calcined in air at 375°C for 5 hours to give the catalyst.

The stainless steel tubular reactor was charged with 6.1 grams of the unsupported $Mo_{16}V_{5.6}Nb_{0.5}Sb_{0.3}Ca_{0.3}$ calcined oxides catalyst and heated in a sand bath thermostated at 255°C. A 50 weight percent aqueous solution of ethanol was fed at a rate of 0.4 ml per hour to a preheater heated in the sand bath at 255°C that was connected to the inlet of the reactor; simultaneously, oxygen, 7 volume percent in helium, was fed to the preheater at 60 ml per minute. The flow of gases from the preheater to the reactor was continued for 2.5 hours at a reactor pressure of 0.069 mbar (100 psig) and then it was arbitrarily stopped. Gaseous reaction effluent was analyzed for oxygen, nitrogen and carbon monoxide by gas chromatography at 65°C using a 3 m by 3 mm column of 5Å molecular sieve 0.177-0.250 mm (60/80 mesh). Carbon dioxide, ethane and ethylene were analyzed using a 1.8 m by 0.3 mm column packed with material sold under the tradename POROPAK Q 0.177 - 0.297 mm (50/80 mesh). The liquid products, acetic acid, water and any unreacted ethanol, were condensed in a cold-trap and were analyzed using a 2 m by 0.3 mm column packed with material sold under the tradename SP-1000 (1%) on trademarked material CARBOPACK B 0.177-0.250 mm (60/80 mesh). Under the reaction conditions, 98 % of the ethanol charged was converted. Selectivity, based on converted ethanol, to acetic acid was 66 mole percent, to ethylene 3 mole present and to ethane 11 mole percent. The balance of the products were carbon dioxide and carbon monoxide. Selectivity to acetic acid plus ethylene was 69 mole percent.

## Example 2

Using the same catalyst, apparatus and procedure described in Example 1, the effect of ethane added to the gaseous feed stream was studied. It was found that selectivity to acetic acid plus ethylene was dramatically improved and less of the carbon oxides were produced.

In this example, a 50 weight percent aqueous solution of ethanol was fed at a rate of 0.4 mL per hour to the preheater while simultaneously feeding a gas mixture containing 6.5 mole percent oxygen, 87 mole percent ethane and 6.5 mole percent nitrogen to the preheater at 60 ml per minute. The flow of gases from the preheater to the reactor was continued for 2.5 hours at a reactor pressure of 100 psig and sand bath temperature of 255°C and then it was arbitrarily stopped. Under these reaction conditions 98% of the ethanol and 4% of the ethane charged reacted. Selectivity, based on converted ethanol plus ethane, to acetic acid was 55 mole percent and to ethylene was 41 mole percent. The balance of the products were carbon dioxide and carbon monoxide. Selectivity to acetic acid plus ethylene was an unexpected high value of 96 mole percent.

## Example 3

A $Mo_{0.68}V_{0.25}Nb_{0.06}$ calcined oxides catalyst was prepared by the following procedure. Ammonium metavanadate (0.145g atom of V) was dissolved in 200 ml of distilled water and stirred for 15 minutes at 70°C. Niobium oxalate (0.035g atom of Nb) was stirred in another 200 ml of 70°C water for 15 minutes and then added to the vanadium-containing solution and the whole was stirred at 70°C for 15 minutes.

Ammonium molybdate (0.4g atom of Mo) was dissolved in 200 ml of 70°C water and added to the prior mixture. The whole mixture was stirred for 15 minutes at 70°C and then evaporated to dryness as rapidly as possible in a stainless steel steam-heated evaporating dish. The resulting solids were ground and sieved to 0.42-0.84 mm (20 to 40 mesh), dried overnight at 120°C and calcined in air at 350°C for 5 hours.

The $Sn_{0.7} Mo_{0.3}$ oxides catalyst was prepared by the procedure described by Ai (J. Catal., 49, 313 (1977)). To stannous chloride dihydrate (210.5 grams) in 2,000 ml of water at 70°C was added 3% aqueous ammonia until precipitation stopped. The solids were washed until chloride-free, slurried in water and the slurry was added with stirring at 80°C to ammonium molybdate tetrahydrate (70.6 grams) dissolved in a minimum of water. The resulting mixture was evaporated to dryness in a steam dish, the solids were sieved to 0.42 - 0.84 mm (20 to 40 mesh), dried overnight at 120°C and then calcined in a flow of air for 5 hours at 500°C.

A stainless steel tubular reactor measuring 1.27 cm inside diameter and 12.7 cm long was charged with an intimate mixture of 4 grams of the MoVNb calcined oxides catalyst and 2 grams of the SnMo oxides catalyst prepared above mixed with 3 $cm^3$ of 0.42-0.84 mm (20 to 40 mesh)quartz chips. Following the procedure and conditions described in Example 1, a 50 weight percent aqueous solution of ethanol was fed at the rate of 0.4 ml per hour. Simultaneously a gas feed of 7 volume percent oxygen, 7 volume percent nitrogen and 86 volume percent helium was fed to the reactor. The reaction was arbitrarily stopped after 3 hours. Ethanol was completely converted in the reactor to acetic acid and carbon oxides. The selectivity to acetic acid from ethanol was 62 mole percent. Oxygen was converted to the extent of 28 mole percent.


## Example 4

Using the same catalyst combination, apparatus and procedure described in Example 3 a 50 weight percent aqueous solution of ethanol was oxidized using a mixture containing 87 volume percent ethane, 6.5 volume percent oxygen and 6.5 volume percent nitrogen as the gas feed. The ethanol was completely converted; conversion of ethane was 4 mole percent and oxygen conversion was 94 mole percent. The products of the reaction were ethylene, acetic acid and carbon oxides. The selectivity to acetic acid from ethanol plus ethane was 52 mole percent and the selectivity to ethylene from ethanol plus ethane was 37 mole percent.


## Example 5

A $Mo_{0.82}V_{0.18}$ oxides on silica catalyst was prepared by the following procedure. Ammonium metavanadate (0.044g atom of V) was dissolved in 100 mL of distilled water at 70°C and stirred 15 minutes. Ammonium molybdate (0.2 g atom of Mo) was dissolved in 100 mL of distilled water, stirred for 15 minutes, added to the vanadium-containing solution, and the whole was stirred for 15 minutes at 70°C. Silica gel (34 g of Cab-O-Sil M-5) and 50 mL of distilled water were added with stirring and then the resulting mixture was evaporated to dryness as rapidly as possible in a stainless steel steam-heated evaporating dish. The resulting solids were sieved, dried overnight at 120°C, and calcined in air at 350°C for 5 hours.

Using the apparatus and procedure described in Example 3, the reactor was charged with 6 grams of the MoV oxides on silica catalyst prepared above. The gas feed was the same as described in Example 3 and the reaction was arbitrarily stopped after 2.5 hours. Ethanol was completely converted in the reactor; oxygen was converted to the extent of 44 mole percent. There were produced acetic acid, ethane and ethylene. The selectivity to acetic acid from ethanol was 73 mole percent. The selectivity to ethane from ethanol was only 7 mole percent and the selectivity to ethylene from ethanol was a low 2 mole percent.


## Example 6

Using the same catalyst, apparatus and procedure described in Example 5 a 50 weight percent aqueous solution of ethanol was oxidized using the same feed gas mixture described in Example 4. The reaction was arbitrarily stopped after 3 hours. Ethanol was completely converted in the reactor; oxygen was converted to the extent of 81 mole percent. There were produced acetic acid and ethylene. The selectivity to acetic acid from ethanol plus ethane was 66 mole percent. The selectivity to ethylene from ethanol plus ethane was 29 mole percent.

**Claims**

1. A process for the conversion of a feed comprising an aliphatic alcohol containing from 2 to 10 carbon atoms - preferably ethanol - and oxygen to selectively produce the alcohol's corresponding acid - preferably acetic acid - **characterized** by
catalytically oxidizing the alcohol at a temperature of from $75^\circ$ C to $500^\circ$ C and a pressure of from 1 to 75 bar in the gas phase in contact with a calcined catalyst represented by the formula:

$$Mo_xV_y \text{ or } Mo_xV_yZ_z \text{ or } Mo_aV_bNb_cSb_dX_e$$

in the form of its mixed oxides, wherein:
Z can be one or more of Li, Na, Be, Mg, Ca, Sr, Ba, Zn, Cd, Hg, Sc, Y, La, Ce, Al, Tl, Ti, Zr, Hf, Pb, Nb, Ta, As, Sb, Bi, Cr, W, U, Te, Fe, Co, Ni and
x is equal to 0.5 to 0.9;
y is equal to 0.1 to 0.4; and
z is equal to 0 to 1
X is at least one of the metals Li, Na, Be, Mg, Ca, Sr, Ba, Zn, Cd, Hg, Sc, Y, La, Ce, Al, Tl, Ti, Zr, Hf, Pb, Ta, As, Bi, Cr, W, U, Te, Fe, Co and Ni and
a is equal to 0.5 to 0.9;
b is equal to 0.1 to 0.4;
c is equal to 0.001 to 0.2;
d is equal to 0.001 to 0.1; and
e is equal to 0.001 to 1.

2. The process as claimed in claim 1, wherein Z is Sb or Sb + Nb.
3. The process as claimed in claim 1, wherein X is Ca.
4. The process as claimed in claims 1 to 3, wherein the temperature is from $200^\circ$ C to $400^\circ$ C and the pressure is from 1 to 30 bar.
5. The process as claimed in claims 1 to 4, wherein the feed additionally contains ethane.